# EUROPÄISCHE PATENTSCHRIFT

(11) **EP 1 670 530 B1**
(45) Veröffentlichungstag und Bekanntmachung des Hinweises auf die Patenterteilung: **14.06.2017**
(21) Anmeldenummer: 04765017.1
(22) Anmeldetag: 09.09.2004
(51) Int. Cl.: A61M 11/06

(54) **INHALATIONSTHERAPIEVORRICHTUNG MIT VENTIL**
INHALATION THERAPY DEVICE COMPRISING A VALVE
DISPOSITIF DE TRAITEMENT PAR INHALATION COMPORTANT UNE SOUPAPE

(30) Priorität: 02.10.2003 DE 10345950
(43) Veröffentlichungstag der Anmeldung: 21.06.2006
(73) Patentinhaber: PARI GmbH Spezialisten für effektive Inhalation, 82319 Starnberg (DE)
(72) Erfinder: KREUTZMANN, Vera, 9450 Altstätten (CH); ROSENBEIGER, Sven, 67685 Weilerbach (DE); MORNHINWEG, Markus, 86911 Diessen (DE); KUMMER, Frank, 84028 Landshut (DE); SELZER, Titus, 80686 München (DE)
(74) Vertreter: Hoffmann Eitle
(86) Internationale Anmeldenummer: PCT/EP2004/010084
(87) Internationale Veröffentlichungsnummer: WO 2005/032630

(56) Entgegenhaltungen:
- WO-A-2004/041336
- DE-C1- 19 902 847
- DE-C1- 19 953 317
- DE-U1- 8 703 534
- US-A- 4 333 450
- US-A- 6 039 042
- US-A1- 2003 037 785
- US-A1- 2004 094 150

## Beschreibung

Die Erfindung betrifft eine Inhalationstherapievorrichtung mit Ventil, insbesondere eine Inhalationstherapievorrichtung mit einem Ventil, die leicht zu reinigen und dabei einfach und sicher in der Handhabung ist.

Inhalationstherapievorrichtungen werden verwendet, um Patienten mit Erkrankungen der Atemwege geeignete Medikamente in Form eines Aerosols zu verabreichen. Durch die Einstellung der Tröpfchengröße mittels einer entsprechenden Auslegung eines Verneblers kann gesteuert werden, an welchen Stellen (Rachenraum, Bronchien, Lungen) das Medikament deponiert werden soll. Dabei atmet der Patient das vernebelte Medikament durch den Mund über ein Mundstück ein, um eine optimale Adaption der Inhalationstherapievorrichtung an den Patienten zu erreichen. Um dem Patienten zu ersparen, zwischen dem Einatmen und dem Ausatmen das Mundstück der Inhalationstherapievorrichtung vom Mund zu entfernen, ist vorgesehen, dass der Patient das vernebelte Medikament nicht nur aus der Inhalationstherapievorrichtung einatmet, sondern auch wieder in die Inhalationstherapievorrichtung hinein ausatmet. Dabei wird der Einatemstrom und der Ausatemstrom in der Regel durch Ventile gesteuert, um die Luftströmung in der Inhalationstherapievorrichtung zu führen. Dazu wird für eine optimale Verabreichung eines Medikaments beim Einatmen und Ausatmen des Patienten die Einatemluft und die Ausatemluft unterschiedlich geführt, damit das Medikament in der Inhalationstherapievorrichtung beim Ausatmen nicht durch Ausatemkondensat verunreinigt wird oder mit dem Ausatemstrom aus der Inhalationstherapievorrichtung geführt wird. Zu diesem Zweck müssen an der Inhalationstherapievorrichtung Ventile vorgesehen sein, um die Luftströmung beim Einatmen und Ausatmen entsprechend zu steuern.

Die Ventile sind bei einer Inhalationstherapievorrichtung in der Regel Verschmutzungen durch Medikamentenreste, Sputum (Speichel) und Ausatemkondensat ausgesetzt. Um den hygienischen Anforderungen zu entsprechen, vor allem bei Verwendung der Inhalationstherapievorrichtung bei unterschiedlichen Patienten, müssen auch die Ventile daher regelmäßig gereinigt werden, um sie von Medikamentenresten, Ausatemkondensat und Sputumresten zu befreien. Zu diesem Zweck sollten die Ventile derart beschaffen sein, dass sie auf einfache Weise eine gute Reinigung ermöglichen. Die Gehäuse und Halterungen von Ventilen weisen jedoch oftmals Stellen auf, die nur mit Mühe gereinigt werden können oder gänzlich unzugänglich und deshalb praktisch nicht zu reinigen sind. Das ist vor allem bei Ventilen der Fall, bei denen das Ventilelement nicht vom Ventilsitz entfernt werden kann, da es sich beispielsweise in einer komplexen Baueinheit befindet oder fest verbunden sind.

Andererseits müssen die Ventile leicht zu befestigen sein, um dem Anwender eine umständliche Handhabung des Gerätes zu ersparen, was vor allem bei Patienten notwendig ist, die im Zusammenhang mit ihrer Atemwegserkrankung körperlich eingeschränkt sind, vor allem sehr alte Menschen.

Weiterhin muss in jedem Fall verhindert werden, dass sich Ventilteile, insbesondere das Ventilelement, von dem Ventil lösen und beim Einatmen vom Patienten verschluckt werden. Da der Patient bei der Inhalationstherapie oftmals tief einatmet, würde ein Verschlucken oder ein Einatmen eines Ventilteils lebensgefährliche Folgen haben, da es mit dem Einatemstrom tief in die Lunge gesogen werden kann.
Im Stand der Technik sind Inhalationstherapievorrichtungen bekannt, die mit einem Ventil ausgestattet sind. Diese Ventile dienen dazu, den Luftstrom in einer Inhalationstherapievorrichtung zu leiten, so dass die Luft entsprechend der Funktion der Inhalationstherapievorrichtung fließt. Ein Aerosolerzeuger gibt ein Aerosol in eine Verneblerkammer ab. Dieses Aerosol wird von dem Einatemluftstrom mitgenommen und beim Patienten durch Einatmen appliziert. Dabei verhindert ein Einatemventil, dass das Aerosol in die Umgebung abgegeben wird, beispielsweise bei fehlender Luftströmung, etwa in Atempausen oder bei Ausatemvorgängen mit umgekehrter Luftströmung, in dem das Ventil nur einen Luftstrom von außen in die Verneblerkammer hinein zulässt. Ebenso werden auch Ausatemventile verwendet, die dazu dienen, den Überdruck beim Ausatmen des Patienten in die Inhalationstherapievorrichtung durch Abgabe der Ausatemluft an die Umgebung abzubauen oder zu verhindern, dass die Ausatemluft durch den Einatemweg strömt. Die Ventile (Einatemventil und Ausatemventil) sollen aber auch verhindern, dass die Ausatemluft fehlgeleitet wird und das Medikament aus der Inhalationstherapievorrichtung mit in die Umgebung genommen wird. Solche Inhalationstherapievorrichtungen sind z.B. aus den Dokumenten US 2003/037785 A1, DE 87 03 534 U1, DE 199 02 847 C1, US 4 333 450 A und DE 199 53 317 C1 bekannt. Das Dokument US 2003/037785 A1 offenbart eine Inhalationstherapievorrichtung gemäß dem Oberbegriff der Ansprüche 1 und 22.

Diese Ventile von Inhalationstherapievorrichtungen im Stand der Technik sind entweder leicht lösbar, enthalten dann jedoch Teile, die vom Patienten bei nicht sachgemäßer Befestigung leicht verschluckt werden können, oder können nur mit viel Mühe und Aufwand zerlegt und zusammengesetzt werden, wodurch die Handhabbarkeit sehr schlecht ist und oftmals auch die Eigenschaft verloren geht, das Ventil einfach reinigen zu können. In der Regel sind die kritischen Stellen bei einem Ventil hinsichtlich einer Verschmutzung im Bereich des Dichtungssitzes oder einer Befestigung aufgrund oft kompakt aufgebauter Ventile nicht für eine Reinigung zugänglich.

Die Aufgabe der vorliegenden Erfindung ist es, die Nachteile der Inhalationstherapievorrichtungen im Stand der Technik zu beseitigen und eine Inhalationstherapievorrichtung bereit zu stellen, die ein Ventil aufweist, das konstruktionsbedingt leicht zu reinigen und dabei in der Handhabung sicher und einfach ist.

Diese Aufgabe wird gelöst durch eine Inhalationstherapievorrichtung gemäß den Merkmalen nach Anspruch 1.

Durch die erfindungsgemäße Ventilelementpositioniereinrichtung wird erreicht, dass das Ventilelement so ausgelegt und angeordnet werden kann, dass es von dem geöffneten Vernebler beabstandet ist. In dieser Stellung kann der Vernebler und auch das Ventil einfach gereinigt werden. In geschlossenem Zustand wirkt die Ventilelementpositioniereinrichtung auf das Ventilelement positionierend ein und bringt es an den Ventilsitz heran, von dem es in geöffnetem Zustand beabstandet ist. An welcher Stelle Ventilsitz, Ventilelement und Ventilelementpositioniereinrichtung an dem Vernebler angebracht sind, kann quasi frei festgelegt werden, solange die erfindungsgemäße Einwirkung der Ventilelementpositioniereinrichtung auf das Ventilelement bei der Überführung der Ventilelementpositioniereinrichtung aus der zweiten in die erste Position realisiert wird.

Die oben angegebene Aufgabe wird auch gelöst, durch eine Inhalationstherapievorrichtung gemäß den Merkmalen nach Anspruch 22.

Durch die erfindungsgemäße alternative Lösung wird auch ohne Ventilelementpositioniereinrichtung erreicht, dass das Ventilelement so ausgelegt und angeordnet werden kann, dass es von dem geöffneten Vernebler beabstandet ist. In dieser Stellung kann der Vernebler und auch das Ventil einfach gereinigt werden. Im geschlossenen Zustand wirkt die entsprechende Befestigung des Ventilelementes auf das Ventilelement auch ohne eine Ventilelementpositioniereinrichtung positionierend ein und bringt es an den Ventilsitz heran, von dem es in geöffnetem Zustand beabstandet ist. An welcher Stelle Ventilsitz und Ventilelement an dem Vernebler angebracht sind, kann quasi frei festgelegt werden, so lange die erfindungsgemäße Einwirkung der Befestigung des Ventilelementes auch ohne die Ventilelementpositioniereinrichtung auf das Ventilelement bei der Überführung des Ventilsitzes bezogen auf das Ventilelement aus der zweiten in die erste Position realisiert wird.

Weitere vorteilhafte Ausgestaltungen ergeben sich aus den Unteransprüchen.

So kann die Beabstandung des Ventilelements durch eine entsprechend gestaltete Fixierung des Ventilelements erzielt werden. Dabei wird die Elastizität des Ventilelements ausgenutzt, das sich von selbst in die beabstandete Stellung bringt, wenn die Ventilelementpositioniereinrichtung nicht auf das Ventilelement einwirkt.

Die Elastizität des Ventilelementes kann jedoch ebenso ausgenutzt werden, wenn keine Ventilelementpositioniereinrichtung vorhanden ist, so dass es sich von selbst in die beabstandete Stellung bringt. Die elastischen Kräfte, die dem Ventilelement innewohnen, können beispielsweise eine geradlinige Ausrichtung des Ventilelements bewirken, das dann die erfindungsgemäße beabstandete Stellung einnimmt

Die Vorspannung wird vorteilhafterweise durch Verbiegen des Ventilelementes erzeugt, wozu der Ventilsitz gekrümmt, abgerundet oder angeschrägt ausgebildet sein kann, um das an dem Ventilsitz positionierte Ventilelement zur Erzeugung der Vorspannung zu verbiegen.

Wo sich das Ventil an der erfindungsgemäßen Inhalationsvorrichtung befindet ist beliebig bestimmbar. Erfingdungsgemäß ist der Abschnitt der Wand, in dem sich die Öffnung und damit das Ventil befindet, gegenüber der Verneblerkammer beweglich. Dabei handelt es sich um einen Deckel, der die Verneblerkammer verschließt. Dieser Deckel kann klappbar an einem feststehenden Teil der Verneblerkammer befestigt sein, wobei die Befestigung mit einem Filmscharnier erfolgen kann, das auch als bistabiles Filmscharnier ausgelegt sein kann.

Besonders vorteilhaft ist eine Ausgestaltung der erfindungsgemäßen Inhalationstherapievorrichtung, bei der Ventilsitz und Ventilelementpositioniereinrichtung einerseits und Ventilelement andererseits aus unterschiedlichen Materialien in einem Teil spritzgegossen sind. Das Ventilelement besteht in der Regel aus einem weicheren Material, beispielsweise aus Silikonkautschuk oder einem thermoplastischen Elastomer, als der Ventilsitz und/oder die Ventilelementpositioniereinrichtung.

Bei einer weiteren vorteilhaften Ausgestaltung der erfindungsgemäßen Inhalationstherapievorrichtung sind der Ventilsitz und das Ventilelement aus unterschiedlichen Materialien in einem Teil spritzgegossen. In diesem Fall besteht das Ventilelement in der Regel aus einem weicheren Material als der Ventilsitz, beispielsweise aus Silikonkautschuk oder einem thermoplastischen Elastomer.

Zur Unterstützung der Abdichtungsfunktion des Ventilelements kann an dem Ventilsitz eine umlaufende Dichtlippe vorgesehen werden.

Zu beachten ist, dass sich das Ventil als Einatemventil oder Ausatemventil einsetzen lässt.

Im Folgenden wird die Erfindung anhand von Ausführungsbeispielen unter Bezugnahme auf die Zeichnungen genauer beschrieben. In den Zeichnungen zeigt:
- Figur 1: eine Inhalationstherapievorrichtung mit einem eingebautem Ventil gemäß einer ersten Ausführungsform der vorliegenden Erfindung;
- Figur 2a: ein Ventil für eine Inhalationstherapievorrichtung gemäß der ersten Ausführungsform der vorliegenden Erfindung in einem geschlossenen, strömungsfreien Zustand;
- Figur 2b: ein Ventil für eine Inhalationstherapievorrichtung gemäß der ersten Ausführungsform der vorliegenden Erfindung in einem geöffneten Zustand;
- Figur 2c: ein Ventil für eine Inhalationstherapievorrichtung gemäß der ersten Ausführungsform der vorliegenden Erfindung in einem Zustand abwärtiger Strömung;
- Figur 3a: ein Ventil für eine Inhalationstherapievorrichtung gemäß einer zweiten Ausführungsform der vorliegenden Erfindung in einem geschlossenen Zustand;
- Figur 3b: ein Ventil für eine Inhalationstherapievorrichtung gemäß der zweiten Ausführungsform der vorliegenden Erfindung in einem geöffneten Zustand;
- Figur 4a: ein Ventil für eine Inhalationstherapievorrichtung gemäß einer dritten Ausführungsform der vorliegenden Erfindung in einem geschlossenen Zustand;
- Figur 4b: ein Ventil für eine Inhalationstherapievorrichtung gemäß der dritten Ausführungsform der vorliegenden Erfindung in einem geöffneten Zustand;
- Figur 5a: ein Ventil für eine Inhalationstherapievorrichtung gemäß einer vierten Ausführungsform der vorliegenden Erfindung in einem geschlossenen Zustand;
- Figur 5b: ein Ventil für eine Inhalationstherapievorrichtung gemäß der vierten Ausführungsform der vorliegenden Erfindung in einem geöffneten Zustand;
- Figur 6a: ein Ventil für eine Inhalationstherapievorrichtung gemäß einer fünften Ausführungsform der vorliegenden Erfindung in einem geschlossenen Zustand;
- Figur 6b: ein Ventil für eine Inhalationstherapievorrichtung gemäß der fünften Ausführungsform der vorliegenden Erfindung in einem geöffneten Zustand;
- Figur 7a: ein Ventil für eine Inhalationstherapievorrichtung gemäß einer sechsten Ausführungsform der vorliegenden Erfindung in einem geschlossenen Zustand;
- Figur 7b: ein Ventil für eine Inhalationstherapievorrichtung gemäß der sechsten Ausführungsform der vorliegenden Erfindung in einem geöffneten Zustand;
- Figur 8a: ein Ventil für eine Inhalationstherapievorrichtung gemäß einer weiteren Ausführungsform der vorliegenden Erfindung mit einem bistabilen Filmscharnier in einem geschlossenen Zustand;
- Figur 8b: ein Ventil für eine Inhalationstherapievorrichtung gemäß der weiteren Ausführungsform der vorliegenden Erfindung mit einem bistabilen Filmscharnier in einem geöffneten Zustand;
- Figur 9a: ein Ventil für eine Inhalationstherapievorrichtung gemäß einer weiteren Ausführungsform der vorliegenden Erfindung, bei der das Ventilelement ohne Vorspannung an dem Ventilsitz anliegt, ohne Luftströmung;
- Figur 9b: ein Ventil für eine Inhalationstherapievorrichtung gemäß der weiteren Ausführungsform der vorliegenden Erfindung, bei der das Ventilelement ohne Vorspannung an dem Ventilsitz anliegt, bei einer Luftströmung in die Inhalationstherapievorrichtung hinein;
- Figur 10a: ein Ventil für eine Inhalationstherapievorrichtung gemäß einer weiteren Ausführungsform der vorliegenden Erfindung ohne eine Ventilelementpositioniereinrichtung, bei dem das Ventilelement an dem feststehenden Teil der Inhalationstherapievorrichtung befestigt ist in einem geschlossenem Zustand;
- Figur 10b: ein Ventil für eine Inhalationstherapievorrichtung gemäß der weiteren Ausführungsform der vorliegenden Erfindung ohne eine Ventilelementpositioniereinrichtung, bei dem das Ventilelement an dem feststehenden Teil der Inhalationstherapievorrichtung befestigt ist in einem geöffneten Zustand;
- Figur 11a: ein Ventil für eine Inhalationstherapievorrichtung gemäß einer weiteren Ausführungsform der vorliegenden Erfindung, bei der das Ventilelement an dem beweglichen Teil der Inhalationstherapievorrichtung befestigt ist in einem geschlossenem Zustand; und
- Figur 11b: ein Ventil für eine Inhalationstherapievorrichtung gemäß der weiteren Ausführungsform der vorliegenden Erfindung, bei der das Ventilelement an dem beweglichen Teil der Inhalationstherapievorrichtung befestigt ist, in einem geöffneten Zustand.

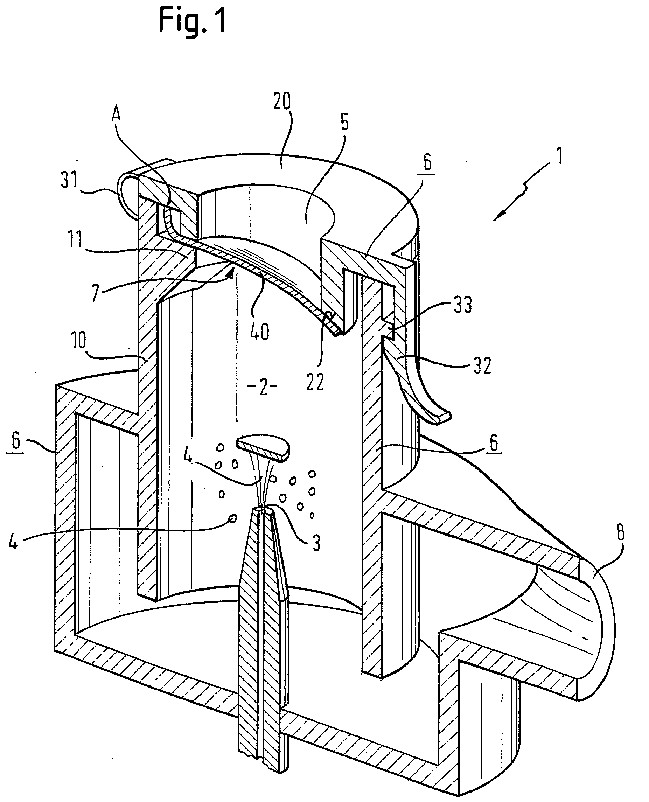

Figur 1 zeigt eine Inhalationstherapievorrichtung 1 gemäß einer Ausführungsform der vorliegenden Erfindung. Die Inhalationstherapievorrichtung 1 weist eine Verneblerkammer 2 auf, in die hinein ein Aerosolerzeuger 3 ein Aerosol 4 erzeugt. Dieses Aerosol 4 wird in der Verneblerkammer 2 bevorratet. Ein erster Abschnitt 10 der Wand 6 der Inhalationstherapievorrichtung 1 ist feststehend; ein zweiter Abschnitt 20 der Wand 6 der Inhalationstherapievorrichtung 1 ist bezogen auf den ersten Abschnitt 10 beweglich. Der bewegliche Abschnitt 20 der Wand 6 ist als Deckel ausgeführt. In dem beweglichen Wandabschnitt 20 ist eine Öffnung 5 vorgesehen, in der ein Ventil 7 angeordnet ist. Das Ventil umfasst einen Ventilsitz 22, ein Ventilelement 40 und eine Ventilelementpositioniereinrichtung 11. In dem in Fig. 1 gezeigten geschlossenen Zustand positioniert die Ventilelementpositioniereinrichtung 11 das Ventilelement 40 derart auf dem Ventilsitz 22, dass das Ventilelement 40 am Ventilsitz 22 leicht vorgespannt anliegt.

Das Ventilelement 40 erhält die Vorspannung beispielsweise dadurch, dass es entlang eines einseitig ansteigenden, gebogenen Ventilsitzes 22 positioniert wird. Das Ventilelement 40 kann, wie in dem in Figur 1 gezeigten Ausführungsbeispiel vorgesehen, fest mit dem Deckel 20 an einer Fixierungsstelle A verbunden sein, um ein Lösen und Verlieren des Ventilelementes zu verhindern. Fertigungstechnisch steht dafür beispielsweise das so genannte 2-K-Verfahren (Zweikomponentenspritzgussverfahren) zur Verfügung, mit dem möglich ist im Spritzguss zwei oder mehrere geeignete, aber unterschiedliche Materialien, aus denen Teile einer Baueinheit bestehen, in einer Baueinheit einteilig zu fertigen. Die Teile der Baueinheit sind dann fest miteinander verbunden und können sich nicht unbeabsichtigt voneinander lösen. Das gewährleistet in dem vorliegenden Fall eine Verliersicherheit und damit auch einen Schutz vor unbeabsichtigtem Einatmen. Bei geeigneter Konstruktion können auch der feststehende Teil 10 der Inhalationstherapievorrichtung 1 zusammen mit dem beweglichen Teil 20 auf diese Weise gefertigt werden.

Das Ventilelement 40 ist vorzugsweise aus einem elastischen Material gefertigt, so dass dann die Verformbarkeit des Ventilelementes 40 die Abdichtung auf dem Ventilsitz 22 gewährleistet. Das elastische Material kann dabei ein Silikonkautschuk oder ein thermoplastisches Elastomer (TPE) sein. Letzterer lässt sich im Zweikomponentenspritzgussverfahren mit weniger elastischen Materialien, wie etwa Polyethylen (PE) oder Polypropylen (PP), gut verarbeiten, so dass die TPE-Teile und die PE- bzw. PP-Teile fest miteinander verbunden sind.

Atmet der Patient bei geschlossenem Deckel 20 beispielsweise durch ein Mundstück 8 ein, so stellt sich durch den Atemzug eine Atemluftströmung ein. Der Unterdruck in der Verneblerkammer 2 bewirkt, dass sich das Ventilelement 40 auslenkt und Luft aus der Umgebung der Inhalationstherapievorrichtung 1 in die Verneblerkammer 2 strömen kann. Der Atemluftstrom durch die Öffnung 5, vorbei an dem ausgelenkten Ventilelement 40 und an dem Aerosolerzeuger 3, nimmt das erzeugte Aerosol 4 mit und gelangt durch das Mundstück 8 zum Patienten.

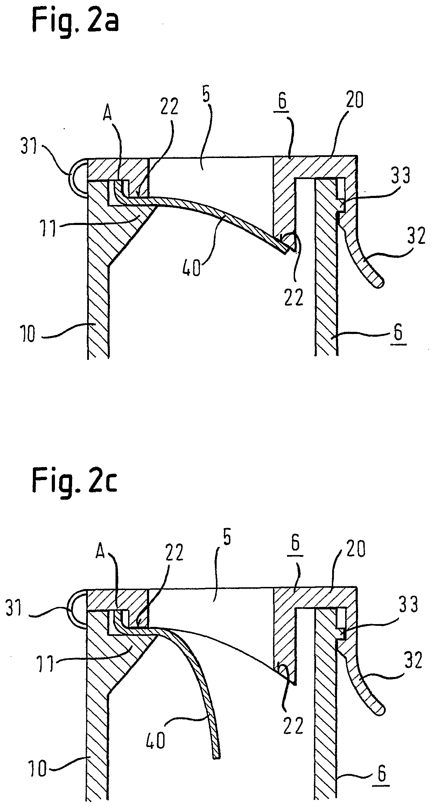

Figur 2a zeigt, wie Figur 1 auch, das Ventil in einem strömungsfreien Zustand. Die Wand 10 der Verneblerkammer 2 der Inhalationstherapievorrichtung 1 wird durch den beweglichen Deckel 20 abgeschlossen, wobei in dem Deckel die Öffnung 5 vorgesehen ist. Die Deckelöffnung 5 ist umgeben von dem Ventilsitz 22 auf dem im geschlossenen Zustand das Ventilelement 40 aufliegt. Dazu hält die Ventilelementpositioniereinrichtung 11 das Ventilelement 40 in einer derartigen Position, dass das Ventilelement 40 auf dem Ventilsitz 22 im strömungsfreien Zustand leicht vorgespannt aufliegt und die Öffnung 5 im Wesentlichen verschließt. Der Deckel ist hier mit einem Filmscharnier 31 an der Verneblerkammerwand 10 der Inhalationstherapievorrichtung 1 befestigt und kann mittels eines Schnappverschlusses 32, 33 in dem geschlossenen Zustand arretiert werden. Der Deckel kann geöffnet werden, wenn die Klinke 32 des Schnappverschlusses von der Raste 33 gelöst wird. Das Ventilelement 40 ist fest mit dem Deckel 20 an der Fixierungsstelle A verbunden, so dass es sich nicht unbeabsichtigt lösen kann. Der Ventilsitz 22 ist derart angeschrägt ausgestaltet, dass das Ventilelement 40 an dem Ventilsitz 22 mit Vorspannung im strömungsfreien Zustand anliegt.

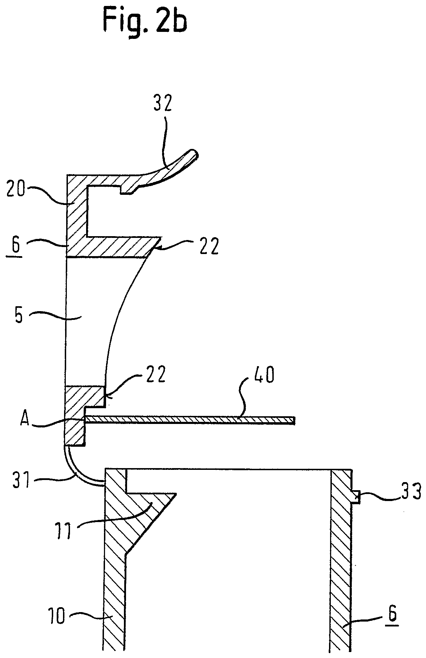

Figur 2a zeigt das Ventil in einem betriebsbereiten geschlossenen Zustand, während Figur 2b das Ventil in einem zur Reinigung geöffneten Zustand zeigt. Da das elastische Ventilelement 40 in diesem Ausführungsbeispiel senkrecht an dem Deckel 20 befestigt, vorzugsweise angeformt ist, bewegt es sich aus der am Ventilsitz 22 anliegenden Position heraus, da die Ventilelementpositioniereinrichtung 11 im Zustand des geöffneten Deckels 20 das Ventilelement 40 nicht mehr mit Vorspannung in der abdichtenden Position auf dem Ventilsitz 22 hält. In dem geöffneten Zustand des Deckels 20 ist das Ventilelement 40 zu dem Ventilsitz 22 erfindungsgemäß derart beabstandet, dass sowohl der Ventilsitz 22, als auch das Ventilelement 40 und die Ventilelementpositioniereinrichtung 11 zugänglich sind und problemlos gereinigt werden können, ohne Kanten oder verdeckte Stellen aufzuweisen, die die Reinigung entsprechend erschweren oder unmöglich machen.

Figur 2c zeigt das Ventil aus Figur 2a in einem Zustand, bei dem Luft durch die Öffnung 5 und damit durch das geöffnete Ventil strömt. Der Luftstrom lenkt das Ventilelement 40 aus, so dass es sich von einem die Vernebelungskammer 2 verschließenden Zustand in einen die Vernebelungskammer 2 nicht verschließenden Zustand auslenkt. Bei einem Aussetzen der Atemluftströmung kehrt das Ventilelement 40 durch die Vorspannung wieder in die Ausgangslage gemäß Figur 2a zurück und liegt dichtend an dem Ventilsitz 22 an.

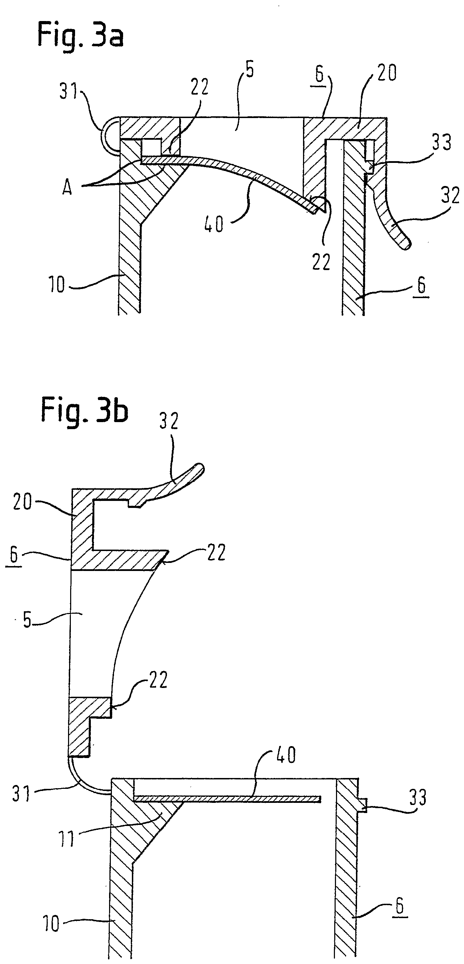

Das Ventilelement 40 muss bei einer Auslegung des Ventils als Einatemventil nicht notwendigerweise an dem Deckel 20 befestigt sein. Vielmehr kann das Ventilelement 40 auch an dem feststehenden Teil 10 der Inhalationstherapievorrichtung 1 befestigt sein oder an der Ventilelementpositioniereinrichtung 11, wie der Figur 3a zu entnehmen ist. Die Fixierungsstelle A kann in diesem Fall an der Wand 10 der Verneblerkammer 2 und/oder an der Ventilelementpositioniereinrichtung 11 vorgesehen sein.

Figur 3b zeigt die in Figur 3a gezeigte Ausführungsform in einem geöffneten Zustand. Vorzugsweise ist das Ventilelement 40 an dem feststehenden Teil 10 der Inhalationstherapievorrichtung 1 in der Nähe der Ventilelementpositioniereinrichtung 11 derart befestigt, dass keine für die Reinigung kritischen Stellen auftreten.

Der Ventilsitz 22 befindet sich auch in dieser Ausführungsform an dem beweglichen Teil, d.h. an dem Deckel 20, der mit einem Filmscharnier 31 an dem feststehenden Teil, d.h. an der Wand 10 der Verneblerkammer 2 der Inhalationstherapievorrichtung befestigt ist.

Die Befestigung des Ventilelements 40 an der Ventilelementpositioniereinrichtung 11 kann auf verschiedene Weise, auch lösbar, erfolgen. Vorzugsweise wird jedoch eine Verbindung durch ein 2-K-Herstellungsverfahren (siehe oben) angewendet, die auf diesem Weg eine besonders vorteilhafte Gestaltung in Bezug auf die Reinhaltung erziehbar ist. Denn an der Verbindungsstelle A zwischen Ventilelement 40 und Ventilelementpositioniereinrichtung 11 wird so eine ganzflächige Verbindung ohne Zwischenraum bzw. ohne Spaltbildung erreicht.

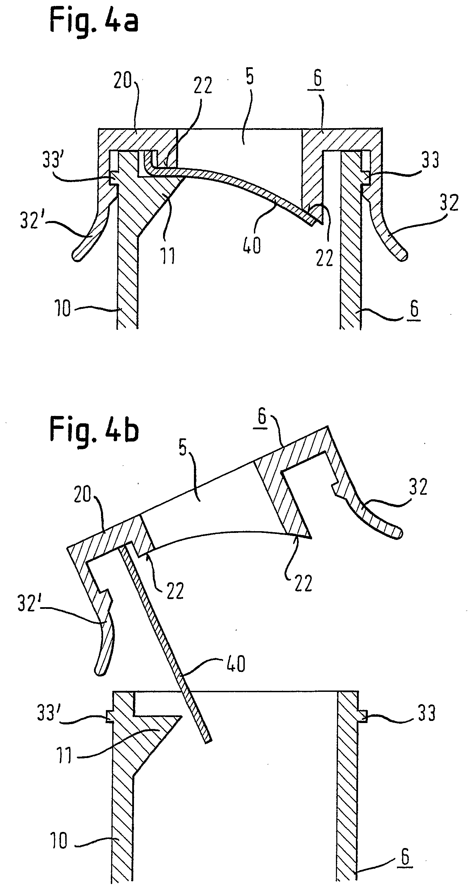

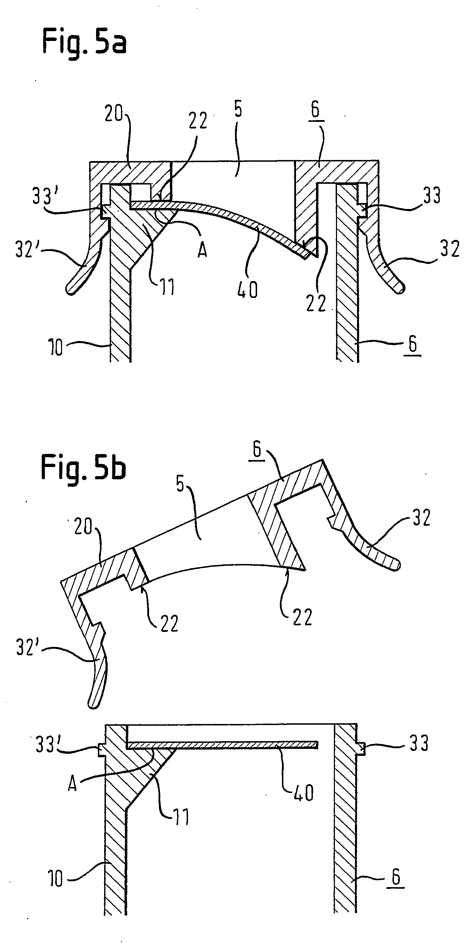

Die Figuren 4a und 4b zeigen eine Ausführungsform, bei der der Deckel 20 nicht mit einem Filmscharnier an der Verneblerkammerwand 10 der Inhalationstherapievorrichtung 1 befestigt ist, sondern mit einem zweiten oder mehreren weiteren Schnappverschlüssen 32', 33' im geschlossenen Zustand an dem feststehenden Teil 10 der Inhalationstherapievorrichtung 1 derart gehaltert ist, dass erfindungsgemäß das Ventilelement 40 durch die Ventilelementpositioniereinrichtung 11 auf dem Ventilsitz 22 positioniert wird. Der Verzicht auf ein Filmscharnier durch den Einsatz weiterer Schnappverbindungen oder - verschlüsse 32', 33' ermöglicht es, den Deckel 20 vollständig von der Inhalationstherapievorrichtung abzunehmen, um diesen gegebenenfalls bei Verschleiß oder nicht entfernbaren Verunreinigungen ersetzen zu können. Auch bei dieser Ausführungsform ist es nicht zwingend notwendig, dass das Ventilelement 40 in der Nähe des Ventilsitzes 22 an dem Deckel 20 befestigt ist. Ebenso kann das Ventilelement 40, wie schon zuvor ausgeführt, an dem feststehenden Teil 10 der Inhalationstherapievorrichtung oder in der Nähe oder an der Ventilelementpositioniereinrichtung 11 derart gehaltert sein, dass es sich nicht unbeabsichtigt löst und keine reinigungskritischen Stellen auftreten, wie die Figur 5a im geschlossenen Zustand und die Figur 5b im geöffneten Zustand zeigt. Diese Ausführungsform ist vorteilhaft, wenn das Ventilelement 40 nicht ausgewechselt werden muss und an den oberen Teil des feststehenden Teils 10 der Inhalationstherapievorrichtung andere Deckel angebracht werden sollen, beispielsweise mit Filtern oder Atemflussbegrenzern versehende Deckel.

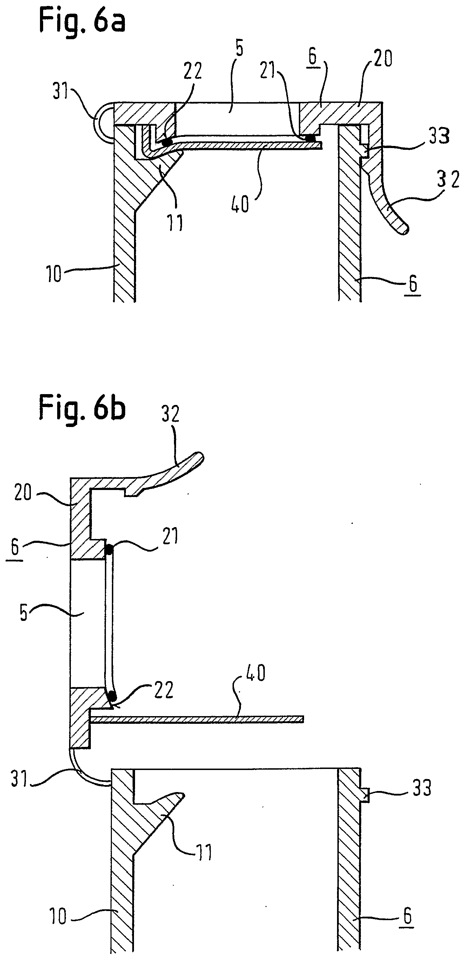

In einer weiteren vorteilhaften Ausführungsform ist der Ventilsitz 22 nicht angeschrägt, sondern im wesentlichen gerade ausgeführt, wie die Figuren 6a und 6b zeigen. Um in diesem Fall eine dichtende Positionierung des Ventilelementes 40 auf dem Ventilsitz 22 mit Vorspannung zu gewährleisten, ist es notwendig die Ventilelementpositioniereinrichtung 11 so zu gestalten, dass durch geeignete Verformung des elastischen Ventilelements 40 eine Vorspannung durch Ausnutzung der Elastizität des Ventilelements erreicht wird. Selbstverständlich kann auch bei dieser Ausführungsform das Ventilelement 40 an dem feststehenden Teil 10 der Inhalationstherapievorrichtung 1 beziehungsweise in der Nähe oder an der Ventilelementpositioniereinrichtung 11 gehaltert sein. In den Figuren 6a und 6b ist weiterhin eine umlaufende Dichtlippe 21 am Ventilsitz 22 gezeigt, welche die abdichtende Wirkung des Ventilelementes 40 auf dem Ventilsitz 22 unterstützt. Die Dichtlippe 21 ist nicht auf die Anwendung bei einem geraden Ventilsitz beschränkt, sondern kann auch bei dem zuvor beschriebenen abgeschrägten oder abgerundeten Ventilsitz vorgesehen werden.

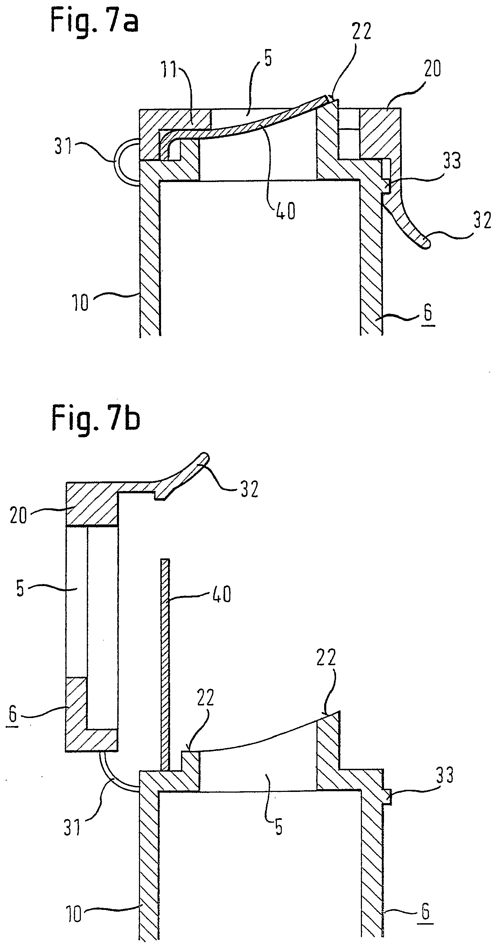

Während die bisher beschriebenen Ausführungsformen sich auf ein Einatemventil beziehen, wird im Folgenden ein Ventil für eine Inhalationstherapievorrichtung gemäß der vorliegenden Erfindung als Ausatemventil beschrieben. Die Figuren 7a und 7b zeigen ein derartiges Ausatemventil in einem geschlossenen und strömungsfreien Zustand beziehungsweise in einem zur Reinigung geöffneten Zustand. Der Ventilsitz 22 befindet sich in dieser Ausführungsform an dem feststehenden Teil 10 der Inhalationstherapievorrichtung. Der Deckel 20 ist mit der Ventilelementpositioniereinrichtung 11 versehen, so dass das Ventilelement 40 im geschlossenen Zustand mit einer Vorspannung auf dem Ventilsitz 22 positioniert wird. Im geöffneten Zustand ist das Ventilelement 40 auf Grund seiner Befestigung an dem feststehenden Teil 10 der Inhalationstherapievorrichtung und seines elastischen Materials von dem Ventilsitz 22 derart beabstandet, dass eine Reinigung sowohl des Ventilelementes 40, als auch des Ventilsitzes 22 problemlos möglich ist, ohne dass reinigungskritische Stellen auftreten.

Wie in den zuvor beschriebenen Ausführungsformen kann auch hier das Ventilelement 40 an bzw. in der Nähe der Ventilelementpositioniereinrichtung 11 befestigt sein.

Ebenfalls kann der Deckel 20 mit weiteren Schnappverbindungen oder Schnappverschlüssen 32, 33 derart befestigt werden, dass die Funktion der Ventilelementpositioniereinrichtung 11 gewährleistet bleibt. Jedoch kann der Deckel 20 dann vollständig von dem feststehenden Teil 10 der Inhalationstherapievorrichtung 1 entfernt werden, um diesen gegebenenfalls auswechseln zu können.

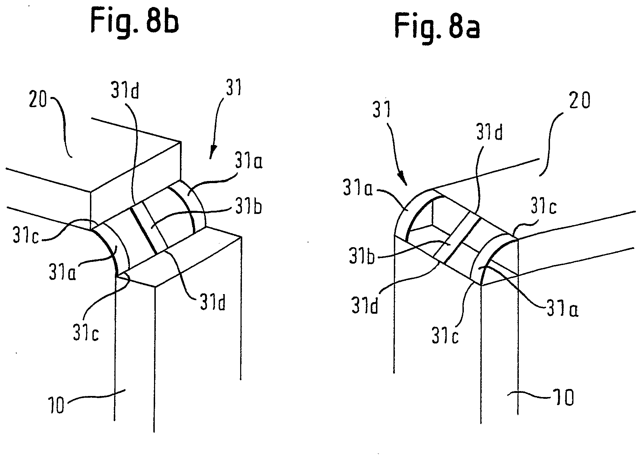

Wie die Figuren 8a und 8b zeigen, kann das einfache Filmscharnier 31, dass den Deckel 20 mit dem feststehenden Teil 10 der Inhalationstherapievorrichtung verbindet, durch ein bistabiles Filmscharnier ersetzt werden. Dieses bistabile Filmscharnier hat im Wesentlichen zwei stabile Positionen, eine im geschlossenen Zustand, wobei der Deckel 20 dann zusätzlich zur Verschlusswirkung des Filmscharniers mit einer Schnappverbindung 32, 33 arretiert werden kann, und eine im geöffneten Zustand. Der stabilisierte geöffnete Zustand gewährleistet, dass der Deckel 20 nicht unbeabsichtigt, beispielsweise während der Reinigung zufällt und somit den Reinigungsvorgang erschwert.

Figur 8a zeigt schematisch ein derartiges Filmscharnier in einem stabilen geschlossenen Zustand und Figur 8b zeigt schematisch ein derartiges Filmscharnier in einem stabilen geöffnetem Zustand. In einer Ausführungsform sind die Elemente 31a dabei gebogen und elastisch und die Elemente 31b gerade und weniger elastisch. Das Teil 31b ist an den Stellen 31d gelenkig mit den Teilen 10 und 20 verbunden, während das Teil 31a an den Stellen 31c starr mit den Teilen 10 und 20 verbunden ist, d.h. der Anstellwinkel zwischen den Oberflächen der Teile 10 bzw. 20 und den Oberflächen der Teile 31a ist dabei nicht veränderlich. Durch das Bewegen von einer geöffneten in eine geschlossene Position muss die Rückstellkraft der Teile 31a überwunden werden, um dabei in die stabile geschlossene Position zu gelangen.

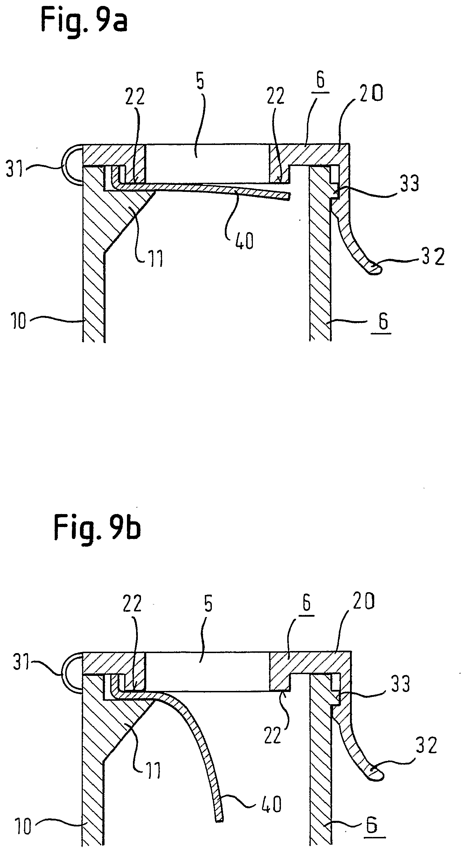

In einer weiteren bevorzugten Ausführungsform kann das Ventilelement 40 auch ohne Vorspannung an dem Ventilsitz 22 anliegen. In einem strömungsfreien Zustand, wie er in Figur 9a gezeigt ist, kann das dazu führen, dass das Ventilelement 40 durch die Schwerkraft in geringem Maße von dem Ventilsitz 22 beabstandet ist, was jedoch die Funktionsfähigkeit des Ventilelementes und des Ventilsitzes nicht beeinträchtigt, da sich das Ventilelement bei einer entsprechenden Strömung auf Grund seines geringen Gewichtes unmittelbar auslenkt und dann an dem Ventilsitz 22 dichtend anliegt. Bei einer entsprechenden Strömung in die Inhalationstherapievorrichtung hinein wird das Ventilelement 40, wie schon zuvor beschrieben, ausgelenkt, so dass das Ventil geöffnet ist (Figur 9b). Die Ventilelementpositioniereinrichtung 11 dient in diesem Fall dazu, das Ventilelement 40 in eine entsprechende Position zu bringen, bei der das Ventilelement 40 zumindest in der Nähe des Ventilsitzes 22 in einem geschlossenen Zustand der Inhalationstherapievorrichtung angeordnet ist. Eine derartige Ausführungsform, bei der das Ventilelement 40 ohne Vorspannung an dem Ventilsitz 22 anliegt ist insbesondere dann vorteilhaft, wenn der Einatemwiderstand reduziert werden soll, da der Patient dann nicht gegen die Vorspannung des Ventilelementes durch das Ventil einatmen muss.

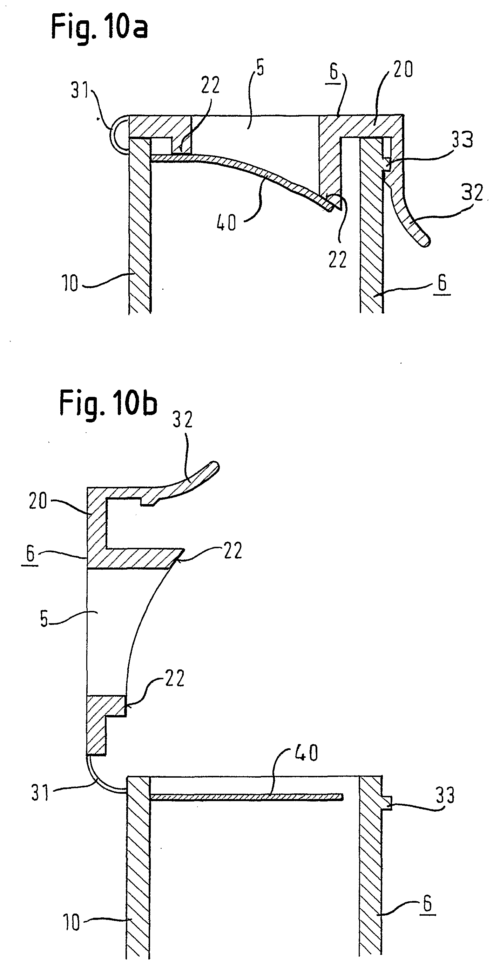

Figur 10a und Figur 10b zeigen ein bevorzugtes Ausführungsbeispiel, bei dem das Ventilelement 40 direkt an dem feststehenden Teil 10 der Inhalationstherapievorrichtung angeformt ist, so dass es ohne eine Ventilelementpositioniereinrichtung an dem Ventilsitz 22 anliegt. In dem hier gezeigten Ausführungsbeispiel in Figur 10a liegt das Ventilelement 40 mit einer Vorspannung an dem Ventilsitz 22 an, es wird jedoch betont, dass das Ventilelement 40 bei dieser Ausführungsform ohne Ventilelementpositioniereinrichtung, bei entsprechender Ausführung des Ventilsitzes auch ohne Vorspannung an dem Ventilsitz anliegen kann. Figur 10b zeigt das bevorzugte Ausführungsbeispiel in einem geöffneten Zustand, bei dem das Ventilelement 40 ohne eine Ventilelementpositioniereinrichtung direkt an dem feststehenden Teil der Inhalationstherapievorrichtung 10 befestigt ist, so dass die Inhalationstherapievorrichtung in geöffnetem Zustand keine Stelle an dem Ventilsitz 22 und dem Ventilelement 40 aufweist, die für eine Reinigung kritisch sein könnte. Dieses Ausführungsbeispiel kann auch derart modifiziert werden, dass der bewegliche Teil 20 der Inhalationstherapievorrichtung 1 mit zwei oder mehr Schnappverbindungen an dem feststehenden Teil 10 der Inhalationstherapievorrichtung 1 befestigt werden kann, wie in einem vorangegangenen Ausführungsbeispiel bereits beschrieben wurde. Dadurch wird eine Austauschbarkeit des Deckels ermöglicht, beispielsweise durch einen gerade ausgeführten Ventilsitz, um die Vorspannung des Ventilelementes 40 auf dem Ventilsitz 22 zu vermindern, beispielsweise um den Einatemwiderstand zu verringern.

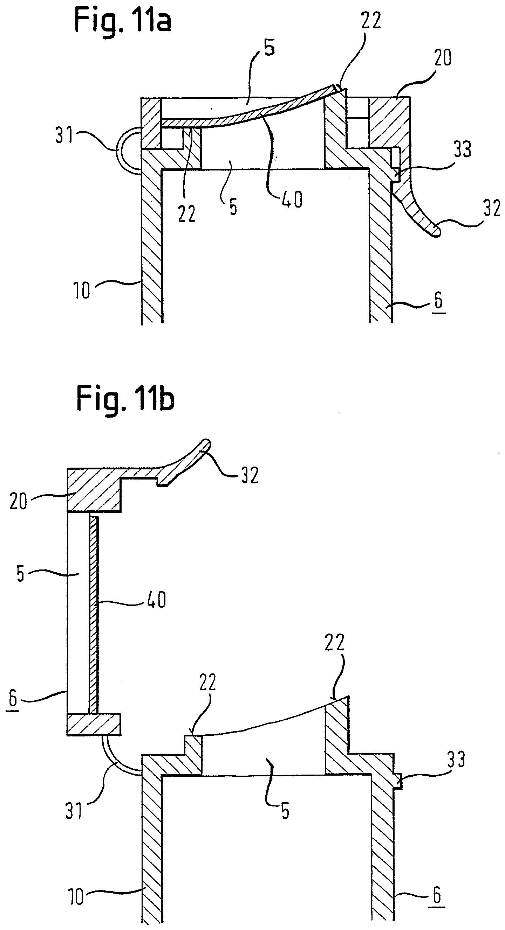

In einer weiteren vorteilhaften Ausführungsform, wie sie in den Figuren 11a und 11b gezeigt ist, ist das Ventilelement an dem beweglichen Teil 20 der Inhalationstherapievorrichtung 1 ohne eine Ventilelementpositioniereinrichtung befestigt. Das Ventilelement ist derart an dem beweglichen Teil 20 der Inhalationstherapievorrichtung befestigt, dass es in dem hier gezeigten Beispiel mit einer Vorspannung an dem Ventilsitz 22 anliegt und die Öffnung 5 der Inhalationstherapievorrichtung verschließt. Die hier gezeigte Ausführungsform kann bei einer Inhalationstherapievorrichtung als Ausatemventil verwendet werden. Durch eine entsprechende Gestaltung des Ventilsitzes 22 kann das Ventilelement 40 auch ohne Vorspannung an dem Ventilsitz 22 anliegen, beispielsweise wenn der Ventilsitz 22 gerade ausgeführt ist. In einem geöffneten Zustand, wie er in Figur 11b gezeigt ist, ist das Ventilelement von dem Ventilsitz 22 derart beabstandet, dass sowohl das Ventilelement 40, als auch der Ventilsitz 22 der Inhalationstherapievorrichtung problemlos gereinigt werden können. Das hier gezeigte Ausführungsbeispiel kann derart modifiziert werden, dass der bewegliche Teil 20 der Inhalationstherapievorrichtung 1 statt mit einem Filmscharnier auch mit zwei oder mehr Schnappverbindungen arretiert werden kann. Das ermöglicht den Austausch des beweglichen Teils und damit den Austausch eines eventuell beschädigten Ventilelementes 40, ohne dass die Inhalationstherapievorrichtung als solche auf Grund eines defekten Ventilelementes 40 unbrauchbar wird.

## Patentansprüche

1. Inhalationstherapievorrichtung (1) mit
- einer Verneblerkammer (2) mit einem feststehenden Wandabschnitt (10) und einem beweglichen Wandabschnitt (20), der bezogen auf den feststehenden Wandabschnitt (10) der Verneblerkammer (2) beweglich ist, wobei der beweglichen Wandabschnitt (20) als Deckel (20) zum Verschließen der Verneblerkammer (2) ausgestaltet ist,
- einem Aerosolerzeuger (3), derart angeordnet, dass er ein erzeugtes Aerosol (4) in die Verneblerkammer (2) abgibt,
- einer Öffnung (5) in einer Wand (6) des beweglichen Wandabschnitts (20) der Verneblerkammer (2), und
- einem Ventil (7), dass an der Öffnung (5) in der Wand (6) des beweglichen Wandabschnitts (20) der Verneblerkammer (2) angeordnet ist,
wobei das Ventil umfasst:
- einen Ventilsitz (22),
- ein elastisches Ventilelement (40), und
- eine Ventilelementpositioniereinrichtung (11), **dadurch gekennzeichnet, dass** das Ventilelement (40) an dem Deckel (20), an dem feststehenden Wandabschnitt (10) oder an der Ventilelementpositioniereinrichtung (11) derart befestigt ist, dass
(i) in einem geöffneten Zustand des Deckels (20),
- das Ventilelement (40) von dem Ventilsitz (22) derart beabstandet ist, dass sowohl der Ventilsitz (22) als auch das Ventilelement (4) und die Ventilelementpositioniereinrichtung (11) für eine Reinigung ohne Kanten oder versteckte Stellen zugänglich sind, wenn das Ventilelement (40) an dem Deckel (20) befestigt ist, oder
- das Ventilelement (40) von dem Ventilsitz (22) derart beabstandet ist, dass eine Reinigung des Ventilelements (40) und des Ventilsitzes ohne Auftreten reinigungskritischer Stellen möglich ist, wenn das Ventilelement (40) an dem feststehenden Wandabschnitt (10) oder an der Ventilelementpositioniereinrichtung (11) befestigt ist; und
(ii) in einem geschlossenen Zustand des Deckels (20),
das Ventilelement (40) in einem strömungsfreien Zustand durch die Ventilelementpositioniereinrichtung (11), die sich auf einer Seite des Ventilelements befindet, auf dem Ventilsitz (22), der sich auf einer entgegengesetzten Seite des Ventilelements befindet, positioniert wird.

2. Inhalationstherapievorrichtung nach Anspruch 1, **dadurch gekennzeichnet, dass** die Beabstandung durch die Fixierung des Ventilelements (40) erzielt wird.

3. Inhalationstherapievorrichtung nach einem der Ansprüche 1 oder 2, **dadurch gekennzeichnet, dass** das Ventilelement (40) in der Nähe oder an der Ventilelementpositioniereinrichtung (11) befestigt ist.

4. Inhalationstherapievorrichtung nach einem der Ansprüche 1 oder 2, **dadurch gekennzeichnet, dass** das Ventilelement (40) in der Nähe oder an dem Ventilsitz (22) befestigt ist.

5. Inhalationstherapievorrichtung nach einem der vorangegangenen Ansprüche, **dadurch gekennzeichnet, dass** das Ventilelement (40) mit einer Vorspannung auf dem Ventilsitz (22) positioniert wird.

6. Inhalationstherapievorrichtung nach Anspruch 5, **dadurch gekennzeichnet, dass** die Vorspannung durch Verbiegen des Ventilelementes (40) erzeugt wird.

7. Inhalationstherapievorrichtung nach Anspruch 6, **dadurch gekennzeichnet, dass** der Ventilsitz (22) krümmt, abgerundet oder angeschrägt ausgebildet ist, um das an dem Ventilsitz positionierte Ventilelement (40) zur Erzeugung der Vorspannung zu verbiegen.

8. Inhalationstherapievorrichtung nach einem der vorangegangenen Ansprüche, **dadurch gekennzeichnet, dass** der beweglichen Wandabschnitt (20), in dem sich die Öffnung (5) befindet, gegenüber der Verneblerkammer (2) beweglich ist.

9. Inhalationstherapievorrichtung nach Anspruch 1, **dadurch gekennzeichnet, dass** der Deckel (20) klappbar an dem feststehenden Wandabschnitt (10) der Verneblerkammer (2) befestigt ist.

10. Inhalationstherapievorrichtung nach Anspruch 8 oder 9, **dadurch gekennzeichnet, dass** der Deckel mit einem Filmscharnier (31) klappbar befestigt ist.

11. Inhalationstherapievorrichtung nach Anspruch 10, **dadurch gekennzeichnet, dass** das Filmscharnier (31) bistabil ist.

12. Inhalationstherapievorrichtung nach einem der Ansprüche 8 bis 11, **dadurch gekennzeichnet, dass** der Ventilsitz (22) andem Deckel, und die Ventilelementpositioniereinrichtung (11) an dem feststehenden wandabschnitt (10) der Verneblerkammer (2) vorgesehen ist.

13. Inhalationstherapievorrichtung nach einem der Ansprüche 8 bis 11, **dadurch gekennzeichnet, dass** die Ventilelementpositioniereinrichtung (11) an dem Deckel, und der Ventilsitz (22) an dem feststehenden Wandabschnitt (10) der Verneblerkammer (2) vorgesehen ist.

14. Inhalationstherapievorrichtung nach einem der vorangegangenen Ansprüche, **dadurch gekennzeichnet, dass** der bewegliche Wandabschnitt (20), in dem geschlossene Zustand mit mindestens einer Schnappverbindung (32, 33) arretierbar ist.

15. Inhalationstherapievorrichtung nach einem der vorangegangenen Ansprüche, wobei wenigstens zwei der Teile Ventilsitz (22), Ventilelement (40) und Ventilelementpositioniereinrichtung (11) in einem Teil spritzgegossen sind.

16. Inhalationstherapievorrichtung nach einem der vorangegangenen Ansprüche, **dadurch gekennzeichnet**, die Teile Ventilsitz (22), Ventilelement (40) und Ventilelementpositioniereinrichtung (11) aus unterschiedlichen Materialien in einem Teil spritzgegossen sind.

17. Inhalationstherapievorrichtung nach einem der vorangegangenen Ansprüche, **dadurch gekennzeichnet, dass** die Teile Ventilsitz (22), Ventilelement (40) und/oder Ventilelementpositioniereinrichtung (11) in einem Zweikomponentenspritzgussverfahren hergestellt sind.

18. Inhalationstherapievorrichtung nach einem der vorangegangenen Ansprüche, **dadurch gekennzeichnet, dass** das Ventilelement (40) im Wesentlichen aus einem weicheren Material als der Ventilsitz (22) und/oder die Ventilelementpositioniereinrichtung (11) besteht.

19. Inhalationstherapievorrichtung nach einem der vorangegangenen Ansprüche, **dadurch gekennzeichnet, dass** das Ventilelement (40) im Wesentlichen aus Silikonkautschuk oder thermoplastischem Elastomer besteht.

20. Inhalationstherapievorrichtung nach einem der vorangegangenen Ansprüche, **dadurch gekennzeichnet, dass** der Ventilsitz (11) ein umlaufende Dichtlippe (21) aufweist.

21. Inhalationstherapievorrichtung nach einem der vorangegangenen Ansprüche, **dadurch gekennzeichnet, dass** das Ventil (7) ein Einatemventil oder ein Ausatemventil ist.

22. Inhalationstherapievorrichtung (1) mit
- einer Verneblerkammer (2) mit einem feststehenden Wandabschnitt (10) und einem beweglichen Wandabschnitt (20), der bezogen auf den feststehenden Wandabschnitt (10) der Verneblerkammer (2) beweglich ist, wobei der beweglichen Wandabschnitt (20) als Deckel (20) zum Verschließen der Verneblerkammer (2) ausgestaltet ist,
- einem Aerosolerzeuger (3), derart angeordnet, dass er ein erzeugtes Aerosol (4) in die Verneblerkammer (2) abgibt,
- einer Öffnung (5) in einer Wand (6) des beweglichen Wandabschnitts (20) der Verneblerkammer (2), und
- einem Ventil (7), dass an der Öffnung (5) in der Wand (6) des beweglichen Wandabschnitts (20) der Verneblerkammer (2) angeordnet ist,
wobei das Ventil umfasst:
- einen Ventilsitz (22), und
- ein elastisches Ventilelement (40), **dadurch gekennzeichnet, dass** das Ventilelement (40) an dem Deckel (20) oder an dem feststehenden Wandabschnitt (10) derart befestigt ist, dass
(i) in einem geöffneten Zustand des Deckels (20),
- das Ventilelement (40) von dem Ventilsitz (22) derart beabstandet ist, dass sowohl der Ventilsitz (22) als auch das Ventilelement (4) für eine Reinigung ohne Kanten oder versteckte Stellen zugänglich sind, wenn das Ventilelement (40) an dem Deckel (20) befestigt ist, oder
- das Ventilelement (40) von dem Ventilsitz (22) derart beabstandet ist, dass eine Reinigung des Ventilelements (40) und des Ventilsitzes ohne Auftreten reinigungskritischer Stellen möglich ist, wenn das Ventilelement (40) an dem feststehenden Wandabschnitt (10) befestigt ist; und
(ii) in einem geschlossenen Zustand des Deckels (20) das Ventilelement (40) in einem strömungsfreien Zustand auf dem Ventilsitz (22) positioniert wird.

23. Inhalationstherapievorrichtung nach Anspruch 22, **dadurch gekennzeichnet, dass** der Deckel (20) klappbar an dem feststehenden wandabschnitt (10) der Verneblerkammer (2) befestigt ist.

24. Inhalationstherapievorrichtung nach Anspruch 23, **dadurch gekennzeichnet, dass** der Deckel (20) mit einem Filmscharnier (31) klappbar befestigt ist.

25. Inhalationstherapievorrichtung nach Anspruch 24, **dadurch gekennzeichnet, dass** das Filmscharnier (31) bistabil ist.

26. Inhalationstherapievorrichtung nach einem der Ansprüche 22 bis 25, **dadurch gekennzeichnet, dass** der Ventilsitz (22) an dem beweglichen Wandabschnitt (20), und das Ventilelement (40) an dem feststehenden Wandabschnitt (10) der Verneblerkammer (2) vorgesehen ist.

27. Inhalationstherapievorrichtung nach einem der Ansprüche 22 bis 25, **dadurch gekennzeichnet, dass** das Ventilelement (40) an dem beweglichen Wandabschnitt (20), und der Ventilsitz (22) an dem feststehenden Wandabschnitt (10) der Verneblerkammer (2) vorgesehen ist.

28. Inhalationstherapievorrichtung nach einem der Ansprüche 22 bis 27, **dadurch gekennzeichnet, dass** das Ventilelement (40) mit einer Vorspannung auf dem Ventilsitz (22) positioniert wird.

29. Inhalationstherapievorrichtung nach Anspruch 28, **dadurch gekennzeichnet, dass** die Vorspannung durch Verbiegen des Ventilelementes (40) erzeugt wird.

30. Inhalationstherapievorrichtung nach Anspruch 28 oder 29, **dadurch gekennzeichnet, dass** der Ventilsitz (22) krümmt, abgerundet oder angeschrägt ausgebildet ist, um das an dem Ventilsitz positionierte Ventilelement (40) zur Erzeugung der Vorspannung zu verbiegen.

31. Inhalationstherapievorrichtung nach einem der Ansprüche 22 bis 30, **dadurch gekennzeichnet, dass** der bewegliche Wandabschnitt (20), in dem geschlossene Zustand mit mindestens einer Schnappverbindung (32, 33) arretierbar ist.

32. Inhalationstherapievorrichtung nach einem der Ansprüche 22 bis 31, **dadurch gekennzeichnet, dass** wenigstens zwei der Teile Ventilsitz (22), Ventilelement (40) und beweglicher Wandabschnitt (20) bzw. feststehender Wandabschnitt (10) in einem Teil spritzgegossen sind.

33. Inhalationstherapievorrichtung nach einem der Ansprüche 22 bis 31, **dadurch gekennzeichnet**, die Teile Ventilsitz (22), Ventilelement (40) und beweglicher Wandabschnitt (20) bzw. feststehender Wandabschnitte (10) aus unterschiedlichen Materialien in einem Teil spritzgegossen sind.

34. Inhalationstherapievorrichtung nach einem der Ansprüche 22 bis 33, **dadurch gekennzeichnet, dass** die Teile Ventilsitz (22), Ventilelement (40) und/oder beweglicher Wandabschnitt (20) bzw. feststehender Wandabschnitt (10) in einem Zweikomponentenspritzgussverfahren hergestellt sind.

35. Inhalationstherapievorrichtung nach einem der Ansprüche 22 bis 34, **dadurch gekennzeichnet, dass** das Ventilelement (40) im Wesentlichen aus einem weicheren Material als der Ventilsitz-(22) besteht.

36. Inhalationstherapievorrichtung nach einem der Ansprüche 22 bis 35, **dadurch gekennzeichnet, dass** das Ventilelement (40) im Wesentlichen aus Silikonkautschuk oder thermoplastischem Elastomer besteht.

37. Inhalationstherapievorrichtung nach einem der Ansprüche 22 bis 36, **dadurch gekennzeichnet, dass** der Ventilsitz (11) ein umlaufende Dichtlippe (21) aufweist.

38. Inhalationstherapievorrichtung nach einem der Ansprüche 22 bis 37, **dadurch gekennzeichnet, dass** das Ventil (7) ein Einatemventil oder ein Ausatemventil ist.

## Claims

1. Inhalation therapy device (1) having
- a nebuliser chamber (2) with a fixed wall section (10) and a movable wall section (20) which is movable relative to the fixed wall section (10) of the nebuliser chamber (2), wherein the movable wall section (20) is designed as a lid (20) for closing the nebuliser chamber (2),
- an aerosol generator (3) arranged in such a way as to emit a generated aerosol (4) into the nebuliser chamber (2),
- an opening (5) in a wall (6) of the movable wall section (20) of the nebuliser chamber (2), and
- a valve (7) which is arranged on the opening (5) in the wall (6) of the movable wall section (20) of the nebuliser chamber (2),
wherein the valve comprises:
- a valve seat (22),
- an elastic valve element (40), and
- a valve element positioning device (11),
**characterised in that** the valve element (40) is attached to the lid (20), to the fixed wall section (10) or to the valve element positioning device (11) in such a way that
(i) in an open state of the lid (20),
- the valve element (40) is spaced apart from the valve seat (22) in such a way that both the valve seat (22) and the valve element (4) and the valve element positioning device (11) are accessible for cleaning without edges or hidden places when the valve element (40) is attached to the lid (20), or
- the valve element (40) is spaced apart from the valve seat (22) in such a way that cleaning of the valve element (40) and valve seat is possible without the occurrence of places critical to cleaning when the valve element (40) is attached to the fixed wall section (10) or to the valve element positioning device (11); and
(ii) in a closed state of the lid (20),
the valve element (40) is positioned in a flow-free state, by the valve element positioning device (11) which is located on one side of the valve element, on the valve seat (22) which is located on an opposite side of the valve element.

2. Inhalation therapy device according to claim 1, **characterised in that** the spacing is obtained by fixing the valve element (40).

3. Inhalation therapy device according to either of claims 1 or 2, **characterised in that** the valve element (40) is attached in the vicinity or on the valve element positioning device (11).

4. Inhalation therapy device according to either of claims 1 or 2, **characterised in that** the valve element (40) is attached in the vicinity or on the valve seat (22).

5. Inhalation therapy device according to any of the preceding claims, **characterised in that** the valve element (40) is positioned with an initial tension on the valve seat (22).

6. Inhalation therapy device according to claim 5, **characterised in that** the initial tension is generated by bending the valve element (40).

7. Inhalation therapy device according to claim 6, **characterised in that** the valve seat (22) is curved, rounded or bevelled in order to bend the valve element (40) positioned on the valve seat to generate the initial tension.

8. Inhalation therapy device according to any of the preceding claims, **characterised in that** the movable wall section (20) in which the opening (5) is located is movable relative to the nebuliser chamber (2).

9. Inhalation therapy device according to claim 1, **characterised in that** the lid (20) is hinged to the fixed wall section (10) of the nebuliser chamber (2).

10. Inhalation therapy device according to claim 8 or 9, **characterised in that** the lid is hinged with a film hinge (31).

11. Inhalation therapy device according to claim 10, **characterised in that** the film hinge (31) is bistable.

12. Inhalation therapy device according to any of claims 8 to 11, **characterised in that** the valve seat (22) is provided on the lid, and the valve element positioning device (11) is provided on the fixed wall section (10) of the nebuliser chamber (2).

13. Inhalation therapy device according to any of claims 8 to 11, **characterised in that** the valve element positioning device (11) is provided on the lid, and the valve seat (22) is provided on the fixed wall section (10) of the nebuliser chamber (2).

14. Inhalation therapy device according to any of the preceding claims, **characterised in that** the movable wall section (20) can be locked in the closed state with at least one snap-fit connection (32, 33).

15. Inhalation therapy device according to any of the preceding claims, wherein at least two of the components of valve seat (22), valve element (40) and valve element positioning device (11) are injection-moulded in one piece.

16. Inhalation therapy device according to any of the preceding claims, **characterised in that** the components of valve seat (22), valve element (40) and valve element positioning device (11) are injection-moulded from different materials in one piece.

17. Inhalation therapy device according to any of the preceding claims, **characterised in that** the components of valve seat (22), valve element (40) and/or valve element positioning device (11) are made by a two-component injection-moulding process.

18. Inhalation therapy device according to any of the preceding claims, **characterised in that** the valve element (40) is essentially made of a softer material than the valve seat (22) and/or the valve element positioning device (11).

19. Inhalation therapy device according to any of the preceding claims, **characterised in that** the valve element (40) is essentially made of silicone rubber or thermoplastic elastomer.

20. Inhalation therapy device according to any of the preceding claims, **characterised in that** the valve seat (11) has a peripheral sealing lip (21).

21. Inhalation therapy device according to any of the preceding claims, **characterised in that** the valve (7) is an inhalation valve or an exhalation valve.

22. Inhalation therapy device (1) having
- a nebuliser chamber (2) with a fixed wall section (10) and a movable wall section (20) which is movable relative to the fixed wall section (10) of the nebuliser chamber (2), wherein the movable wall section (20) is designed as a lid (20) for closing the nebuliser chamber (2),
- an aerosol generator (3) arranged in such a way as to emit a generated aerosol (4) into the nebuliser chamber (2),
- an opening (5) in a wall (6) of the movable wall section (20) of the nebuliser chamber (2), and
- a valve (7) which is arranged on the opening (5) in the wall (6) of the movable wall section (20) of the nebuliser chamber (2),
wherein the valve comprises:
- a valve seat (22) and
- an elastic valve element (40),
**characterised in that** the valve element (40) is attached to the lid (20) or to the fixed wall section (10) in such a way that
(i) in an open state of the lid (20),
- the valve element (40) is spaced apart from the valve seat (22) in such a way that both the valve seat (22) and the valve element (4) are accessible for cleaning without edges or hidden places when the valve element (40) is attached to the lid (20), or
- the valve element (40) is spaced apart from the valve seat (22) in such a way that cleaning of the valve element (40) and valve seat is possible without the occurrence of places critical to cleaning when the valve element (40) is attached to the fixed wall section (10); and
(ii) in a closed state of the lid (20),
the valve element (40) is positioned in a flow-free state on the valve seat (22).

23. Inhalation therapy device according to claim 22, **characterised in that** the lid (20) is hinged to the fixed wall section (10) of the nebuliser chamber (2).

24. Inhalation therapy device according to claim 23, **characterised in that** the lid (20) is hinged with a film hinge (31).

25. Inhalation therapy device according to claim 24, **characterised in that** the film hinge (31) is bistable.

26. Inhalation therapy device according to any of claims 22 to 25, **characterised in that** the valve seat (22) is provided on the movable wall section (20) of the nebuliser chamber (2), and the valve element (40) is provided on the fixed wall section (10).

27. Inhalation therapy device according to any of claims 22 to 25, **characterised in that** the valve element (40) is provided on the movable wall section (20) of the nebuliser chamber (2), and the valve seat (22) is provided on the fixed wall section (10).

28. Inhalation therapy device according to any of claims 22 to 27, **characterised in that** the valve element (40) is positioned with an initial tension on the valve seat (22).

29. Inhalation therapy device according to claim 28, **characterised in that** the initial tension is generated by bending the valve element (40).

30. Inhalation therapy device according to claim 28 or 29, **characterised in that** the valve seat (22) is curved, rounded or bevelled in order to bend the valve element (40) positioned on the valve seat to generate the initial tension.

31. Inhalation therapy device according to any of claims 22 to 30, **characterised in that** the movable wall section (20) can be locked in the closed state with at least one snap-fit connection (32, 33).

32. Inhalation therapy device according to any of claims 22 to 31, **characterised in that** at least two of the components of valve seat (22), valve element (40) and movable wall section (20) or fixed wall section (10) are injection-moulded in one piece.

33. Inhalation therapy device according to any of claims 22 to 31, **characterised in that** the components of valve seat (22), valve element (40) and movable wall section (20) or fixed wall section (10) are injection-moulded from different materials in one piece.

34. Inhalation therapy device according to any of claims 22 to 33, **characterised in that** the components of valve seat (22), valve element (40) and/or movable wall section (20) or fixed wall section (10) are made by a two-component injection-moulding process.

35. Inhalation therapy device according to any of claims 22 to 34, **characterised in that** the valve element (40) is essentially made of a softer material than the valve seat (22) and/or the valve element positioning device (11).

36. Inhalation therapy device according to any of claims 22 to 35, **characterised in that** the valve element (40) is essentially made of silicone rubber or thermoplastic elastomer.

37. Inhalation therapy device according to any of claims 22 to 36, **characterised in that** the valve seat (11) has a peripheral sealing lip (21).

38. Inhalation therapy device according to any of claims 22 to 37, **characterised in that** the valve (7) is an inhalation valve or an exhalation valve.

## Revendications

1. Dispositif de traitement par inhalation (1) avec
- une chambre de nébuliseur (2) avec une partie de paroi (10) immobile et avec une partie de paroi (20) mobile, qui est mobile par rapport à la partie de paroi (10) immobile de la chambre de nébuliseur (2), dans lequel la partie de paroi (20) mobile est configurée sous la forme d'un couvercle (20) pour fermer la chambre de nébuliseur (2),
- un producteur d'aérosol (3), agencé de telle manière qu'il délivre dans la chambre de nébuliseur (2) un aérosol (4) produit,
- une ouverture (5) dans une paroi (6) de la partie de paroi (20) mobile de la chambre de nébuliseur (2), et
- une valve (7), qui est agencée au niveau de l'ouverture (5) dans la paroi (6) de la partie de paroi (20) mobile de la chambre de nébuliseur (2),
dans lequel la valve comprend :
- un siège de valve (22),
- un élément de valve (40) élastique, et
- un système de positionnement d'élément de valve (11),
**caractérisé en ce que**
l'élément de valve (40) est fixé au niveau du couvercle (20), au niveau de la partie de paroi (10) immobile ou au niveau du système de positionnement d'élément de valve (11) de telle manière que
(i) lorsque le couvercle (20) est à l'état ouvert,
- l'élément de valve (40) est à une distance telle du siège de valve (22) qu'aussi bien le siège de valve (22) que l'élément de valve (4) et le système de positionnement d'élément de valve (11) sont accessibles pour un nettoyage sans arêtes ni endroits cachés lorsque l'élément de valve (40) est fixé au niveau du couvercle (20), ou
- l'élément de valve (40) est à une distance telle du siège de valve (22) qu'un nettoyage de l'élément de valve (40) et du siège de valve est possible sans apparition d'endroits critiques pour le nettoyage lorsque l'élément de valve (40) est fixé au niveau de la partie de paroi (10) immobile ou au niveau du système de positionnement d'élément de valve (11) ; et
(ii) lorsque le couvercle (20) est à l'état fermé,
l'élément de valve (40) est positionné dans un état sans écoulement par le système de positionnement d'élément de valve (11), qui se trouve sur un côté de l'élément de valve, sur le siège de valve (22), qui se trouve sur un côté opposé de l'élément de valve.

2. Dispositif de traitement par inhalation selon la revendication 1, **caractérisé en ce que** l'espacement est obtenu par la fixation de l'élément de valve (40).

3. Dispositif de traitement par inhalation selon l'une des revendications 1 ou 2, **caractérisé en ce que** l'élément de valve (40) est fixé à proximité du ou au niveau du système de positionnement d'élément de valve (11).

4. Dispositif de traitement par inhalation selon l'une des revendications 1 ou 2, **caractérisé en ce que** l'élément de valve (40) est fixé à proximité du ou au niveau du siège de valve (22).

5. Dispositif de traitement par inhalation selon l'une des revendications précédentes, **caractérisé en ce que** l'élément de valve (40) est positionné avec une précontrainte sur le siège de valve (22).

6. Dispositif de traitement par inhalation selon la revendication 5, **caractérisé en ce que** la précontrainte est produite par une déformation de l'élément de valve (40).

7. Dispositif de traitement par inhalation selon la revendication 6, **caractérisé en ce que** le siège de valve (22) est réalisé de manière courbée, arrondie ou biseautée pour déformer l'élément de valve (40) positionné au niveau du siège de valve afin de produire la précontrainte.

8. Dispositif de traitement par inhalation selon l'une des revendications précédentes, **caractérisé en ce que** la partie de paroi (20) mobile, dans laquelle se trouve l'ouverture (5), est mobile par rapport à la chambre de nébuliseur (2).

9. Dispositif de traitement par inhalation selon la revendication 1, **caractérisé en ce que** le couvercle (20) est fixé de manière rabattable au niveau de la partie de paroi (10) immobile de la chambre de nébuliseur (2).

10. Dispositif de traitement par inhalation selon la revendication 8 ou 9, **caractérisé en ce que** le couvercle est fixé de manière rabattable avec une charnière à film (31).

11. Dispositif de traitement par inhalation selon la revendication 10, **caractérisé en ce que** la charnière à film (31) est bistable.

12. Dispositif de traitement par inhalation selon l'une des revendications 8 à 11, **caractérisé en ce que** le siège de valve (22) est prévu au niveau du couvercle, et le système de positionnement d'élément de valve (11) est prévu au niveau de la partie de paroi (10) immobile de la chambre de nébuliseur (2).

13. Dispositif de traitement par inhalation selon l'une des revendications 8 à 11, **caractérisé en ce que** le système de positionnement d'élément de valve (11) est prévu au niveau du couvercle, et le siège de valve (22) est prévu au niveau de la partie de paroi (10) immobile de la chambre de nébuliseur (2).

14. Dispositif de traitement par inhalation selon l'une des revendications précédentes, **caractérisé en ce que** la partie de paroi (20) mobile peut être bloquée dans l'état fermé avec au moins un assemblage par encliquetage (32, 33).

15. Dispositif de traitement par inhalation selon l'une des revendications précédentes, dans lequel au moins deux des pièces parmi le siège de valve (22), l'élément de valve (40) et le système de positionnement d'élément de valve (11) sont moulées par injection en une seule pièce.

16. Dispositif de traitement par inhalation selon l'une des revendications précédentes, **caractérisé en ce que** les pièces parmi le siège de valve (22), l'élément de valve (40) et le système de positionnement d'élément de valve (11) sont moulées par injection en une seule pièce à partir de matériaux différents.

17. Dispositif de traitement par inhalation selon l'une des revendications précédentes, **caractérisé en ce que** les pièces parmi le siège de valve (22), l'élément de valve (40) et/ou le système de positionnement d'élément de valve (11) sont fabriquées dans un procédé de moulage par injection à deux composants.

18. Dispositif de traitement par inhalation selon l'une des revendications précédentes, **caractérisé en ce que** l'élément de valve (40) est constitué sensiblement d'un matériau plus mou que le siège de valve (22) et/ou que le système de positionnement d'élément de valve (11).

19. Dispositif de traitement par inhalation selon l'une des revendications précédentes, **caractérisé en ce que** l'élément de valve (40) est constitué sensiblement de caoutchouc silicone ou d'un élastomère thermoplastique.

20. Dispositif de traitement par inhalation selon l'une des revendications précédentes, **caractérisé en ce que** le siège de valve (11) présente une lèvre d'étanchéité (21) périphérique.

21. Dispositif de traitement par inhalation selon l'une des revendications précédentes, **caractérisé en ce que** la valve (7) est une valve d'inspiration ou une valve d'expiration.

22. Dispositif de traitement par inhalation (1) avec
- une chambre de nébuliseur (2) avec une partie de paroi (10) immobile et avec une partie de paroi (20) mobile, qui est mobile par rapport à la partie de paroi (10) immobile de la chambre de nébuliseur (2), dans lequel la partie de paroi (20) mobile est configurée sous la forme d'un couvercle (20) pour fermer la chambre de nébuliseur (2),
- un producteur d'aérosol (3), agencé de telle manière qu'il délivre dans la chambre de nébuliseur (2) un aérosol (4) produit,
- une ouverture (5) dans une paroi (6) de la partie de paroi (20) mobile de la chambre de nébuliseur (2), et
- une valve (7), qui est agencée au niveau de l'ouverture (5) dans la paroi (6) de la partie de paroi (20) mobile de la chambre de nébuliseur (2),
dans lequel la valve comprend :
- un siège de valve (22), et
- un élément de valve (40) élastique,
**caractérisé en ce que**
l'élément de valve (40) est fixé au niveau du couvercle (20) ou au niveau de la partie de paroi (10) immobile de telle manière que
(i) lorsque le couvercle (20) est à l'état ouvert,
- l'élément de valve (40) est à une distance telle du siège de valve (22) qu'aussi bien le siège de valve (22) que l'élément de valve (4) sont accessibles pour un nettoyage sans arêtes ni endroits cachés lorsque l'élément de valve (40) est fixé au niveau du couvercle (20), ou
- l'élément de valve (40) est à une distance telle du siège de valve (22) qu'un nettoyage de l'élément de valve (40) et du siège de valve est possible sans apparition d'endroits critiques pour le nettoyage lorsque l'élément de valve (40) est fixé au niveau de la partie de paroi (10) immobile ; et
(ii) lorsque le couvercle (20) est à l'état fermé,
l'élément de valve (40) est positionné dans un état sans écoulement sur le siège de valve (22).

23. Dispositif de traitement par inhalation selon la revendication 22, **caractérisé en ce que** le couvercle (20) est fixé de manière rabattable au niveau de la partie de paroi (10) immobile de la chambre de nébuliseur (2).

24. Dispositif de traitement par inhalation selon la revendication 23, **caractérisé en ce que** le couvercle (20) est fixé de manière rabattable avec une charnière à film (31).

25. Dispositif de traitement par inhalation selon la revendication 24, **caractérisé en ce que** la charnière à film (31) est bistable.

26. Dispositif de traitement par inhalation selon l'une des revendications 22 à 25, **caractérisé en ce que** le siège de valve (22) est prévu au niveau de la partie de paroi (20) mobile, et l'élément de valve (40) est prévu au niveau de la partie de paroi (10) immobile de la chambre de nébuliseur (2).

27. Dispositif de traitement par inhalation selon l'une des revendications 22 à 25, **caractérisé en ce que** l'élément de valve (40) est prévu au niveau de la partie de paroi (20) mobile, et le siège de valve (22) est prévu au niveau de la partie de paroi (10) immobile de la chambre de nébuliseur (2).

28. Dispositif de traitement par inhalation selon l'une des revendications 22 à 27, **caractérisé en ce que** l'élément de valve (40) est positionné avec une précontrainte sur le siège de valve (22).

29. Dispositif de traitement par inhalation selon la revendication 28, **caractérisé en ce que** la précontrainte est produite par une déformation de l'élément de valve (40).

30. Dispositif de traitement par inhalation selon la revendication 28 ou 29, **caractérisé en ce que** le siège de valve (22) est réalisé de manière courbée, arrondie ou biseautée pour déformer l'élément de valve (40) positionné au niveau du siège de valve afin de produire la précontrainte.

31. Dispositif de traitement par inhalation selon l'une des revendications 22 à 30, **caractérisé en ce que** la partie de paroi (20) mobile peut être bloquée dans l'état fermé avec au moins un assemblage par encliquetage (32, 33).

32. Dispositif de traitement par inhalation selon l'une des revendications 22 à 31, **caractérisé en ce qu'**au moins deux des pièces parmi le siège de valve (22), l'élément de valve (40) et la partie de paroi (20) mobile ou la partie de paroi (10) immobile sont moulées par injection en une seule pièce.

33. Dispositif de traitement par inhalation selon l'une des revendications 22 à 31, **caractérisé en ce que** les pièces parmi le siège de valve (22), l'élément de valve (40) et la partie de paroi (20) mobile ou la partie de paroi (10) immobile sont moulées par injection en une seule pièce à partir de matériaux différents.

34. Dispositif de traitement par inhalation selon l'une des revendications 22 à 33, **caractérisé en ce que** les pièces parmi le siège de valve (22), l'élément de valve (40) et/ou la partie de paroi (20) mobile ou la partie de paroi (10) immobile sont fabriquées dans un procédé de moulage par injection à deux composants.

35. Dispositif de traitement par inhalation selon l'une des revendications 22 à 34, **caractérisé en ce que** l'élément de valve (40) est constitué sensiblement d'un matériau plus mou que le siège de valve (22).

36. Dispositif de traitement par inhalation selon l'une des revendications 22 à 35, **caractérisé en ce que** l'élément de valve (40) est constitué sensiblement de caoutchouc silicone ou d'un élastomère thermoplastique.

37. Dispositif de traitement par inhalation selon l'une des revendications 22 à 36, **caractérisé en ce que** le siège de valve (11) présente une lèvre d'étanchéité (21) périphérique.

38. Dispositif de traitement par inhalation selon l'une des revendications 22 à 37, **caractérisé en ce que** la valve (7) est une valve d'inspiration ou une valve d'expiration.
